Europäisches Patentamt

European Patent Office

Office européen des brevets

(11) Numéro de publication: **0 169 784**
A2

## (12) DEMANDE DE BREVET EUROPEEN

(21) Numéro de dépôt: 85401484.2

(22) Date de dépôt: 18.07.85

(51) Int. Cl.⁴: **A 61 B 17/22**

(30) Priorité: 20.07.84 IT 2198284

(43) Date de publication de la demande:
29.01.86 Bulletin 86/5

(84) Etats contractants désignés:
AT BE CH DE FR GB LI LU NL SE

(71) Demandeur: SYNTHELABO
58 rue de la Glacière
F-75013 Paris(FR)

(72) Inventeur: Dormia, Enrico
35, Via Belvedere
Lecco(IT)

(74) Mandataire: Bonnetat, Christian et al,
Cabinet PROPI Conseils 23 rue de Léningrad
F-75008 Paris(FR)

(54) **Instrument pour l'extraction de corps étrangers dans les canaux physiologiques de l'organisme.**

(57) – L'invention concerne un instrument pour l'extraction de corps étrangers à partir de canaux physiologiques de l'organisme.

– L'instrument comporte un cathéter (2) comportant un câble intérieur (3) aboutissant à un curseur (4) sur lequel est monté un panier extérieur (5) provoquant l'écartement des parois du canal physiologique et ce panier extérieur (5) contient intérieurement un panier (7) pour l'extraction du corps étranger (10) ; le panier d'écartement (5) maintient ainsi à distance les parois du canal physiologique par rapport aux aspérités (10a) du corps à extraire ainsi empêchées de léser ces parois.

– Application à un cathéter pour l'extraction de calculs dans les canaux physiologiques.

Fig. 2

# Instrument pour l'extraction de corps étrangers dans les canaux physiologiques de l'organisme. 0169784

La présente invention concerne un instrument pour l'extraction de corps étrangers dans les canaux physiologiques de l'organisme.

De tels corps étrangers peuvent consister par exemple dans des concrétions calcaires (calculs) ou analogues. On connaît déjà un instrument du type spécifié ci-dessus et constitué essentiellement d'un tube externe ou cathéter en matière flexible à l'intérieur duquel est monté un câble fin supportant la partie opérationnelle de l'instrument, cette dernière étant constituée d'une pluralité d'éléments filiformes élastiques, avantageusement en acier inoxydable. Ces éléments filiformes sont fixés à leurs extrémités proximales à l'extrémité distale dudit câble. Ces éléments sont assemblés entre eux au niveau de leurs extrémités distales, avantageusement à l'aide d'un embout ou ogive de profil arrondi. Avant leur réunion par une extrémité distale commune comme mentionné ci-dessus, ces éléments filiformes élastiques, dans la position opérationnelle du cathéter, suivent une torsade, par exemple hélicoïdale, partant de la soudure de l'extrémité distale desdits fils métalliques ; ainsi, ces derniers suivent une conformation élargie constituant un panier dans la position ouverte du cathéter. Le nombre des éléments filiformes, comme leur conformation exacte ou leur profil, peut varier à volonté, dès lors que ces éléments filiformes conforment un panier.

En cours d'utilisation, la partie qui constitue le panier est d'abord introduite à l'intérieur du cathéter et ce dernier est engagé dans la zone où les calculs ou autres corps étrangers peuvent se trouver. A ce niveau, le panier est remis en position déployée et les fils élastiques viennent porter contre les parois du canal en fonction de la largeur de ce dernier et ensuite le panier est ramené en arrière en direction du corps étranger de façon à l'emprisonner et à permettre l'entraînement du corps étranger en vue de son extraction.

En pratique, on a constaté que le fonctionnement de ce type d'appareil n'est pas exempt d'inconvénients. En fait en raison de la forme irrégulière des corps étrangers qui peuvent par exemple comporter des aspérités ou des excroissances pointues, on constate que ces corps étrangers, qui dans le mouvement d'extraction viennent se positionner dans la zone distale du panier, débordent au delà de cette zone par leurs parties pointues à travers les fils du panier et viennent au contact de la paroi du canal considéré ; dans ces conditions, les parties pointues des corps étrangers, lors du déplacement de ces derniers, peuvent léser et entailler la paroi intérieure du canal, ce qui nécessite alors une intervention chirurgicale.

La présente invention vise à créer un instrument du type spécifié ci-dessus permettant une extraction de corps étrangers sans agression de la paroi du canal et empêchant que les aspérités des corps étrangers puissent venir au contact de la paroi intérieure du canal au cours de la manoeuvre de l'instrument.

L'invention concerne donc un instrument destiné à l'extraction de corps étrangers depuis les canaux physiologiques de l'organisme et du type comportant un tube ou cathéter récepteur d'un câble de commande relié à son extrémité distale à un panier d'écartement formé de fils élastiques, cet instrument étant caractérisé par le fait que :

a) à l'intérieur du panier d'écartement est prévu un panier d'extraction conformé de façon analogue ;

b) le panier intérieur d'extraction est relié par son extrémité proximale à l'extrémité proximale du panier d'écartement et l'extrémité distale du panier d'extraction est indépendante et écartée de l'extrémité distale du panier extérieur d'écartement ; et

c) entre le panier intérieur d'extraction et le panier extérieur d'écartement se trouve ainsi délimité un volume intermédiaire apte à contenir les aspérités ou excroissances des corps étrangers à extraire.

Ainsi, lors de la mise en oeuvre de l'instrument selon l'invention à l'occasion d'une opération d'extraction, la paroi interne du canal physiologique ne peut en aucune façon se trouver lésée ou entaillée par les aspérités ou saillies d'un corps étranger qui est entraîné ; en effet, ce corps étranger se trouve emprisonné dans le panier intérieur et dans ces conditions les aspérités ou saillies du corps étranger viennent se loger dans le volume intermédiaire défini entre la surface virtuelle d'enveloppement du panier intérieur et celle du panier extérieur, sans causer de dommages à la paroi interne du canal physiologique.

En pratique, on évite ainsi les lésions avec certitude en prévoyant une longueur du panier intérieur correspondant environ à la moitié de la longueur du panier extérieur. En ce qui concerne les sens de torsion en spirale du panier extérieur et de celle du panier intérieur, les torsions pourront être en concordance comme elles pourront être prévues avec un enroulement en sens contraire. De même, les nombres de fils du panier d'écartement et du panier d'extraction peuvent être égaux ou différents. Les sections des fils des deux paniers peuvent être différentes.

La conformation de l'appareil selon l'invention n'entraîne aucun encombrement supplémentaire et l'appareil selon l'invention pourra présenter les mêmes dimensions d'encombrement que les instruments actuellement connus.

L'appareil selon l'invention présente également des avantages du point de vue constructif ; en effet la construction a lieu selon les mêmes normes qui ont été définies pour les instruments traditionnels.

Avantageusement, la zone intermédiaire prévue entre la surface d'enveloppement virtuelle des paniers présente un volume maximum au niveau de la partie distale du panier intérieur.

D'autres caractéristiques et avantages ou détails de l'instrument selon l'invention pour l'extraction de corps étrangers, apparaîtront de la description qui suit en se référant aux dessins annexés dans lesquels :

- la figure 1 montre une vue en coupe longitudinale à travers un canal physiologique dans lequel est engagé un appareil d'extraction de corps étrangers de type connu.

- la figure 2 montre une vue en coupe longitudinale analogue à la figure 1 et présentant un appareil d'extraction de corps étrangers selon l'invention.

- la figure 3 montre une section en coupe transversale de la figure 2, au niveau du corps étranger à extraire.

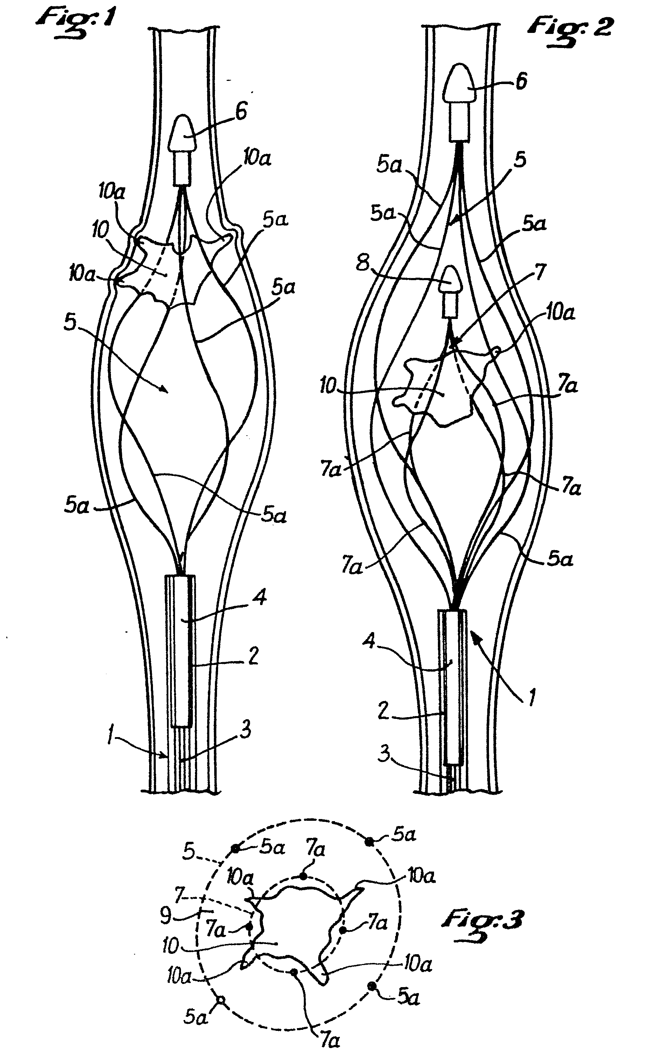

En se référant aux figures dans lesquelles les mêmes pièces ou organes comportent les mêmes références, l'instrument d'extraction de corps étrangers est indiqué sous la référence générale 1. En considérant la figure 1 on voit que l'appareil est constitué d'un cathéter ou analogue 2 contenant intérieurement un câble fin 3 dont l'extrémité supérieure aboutit à un curseur 4. Au sommet de ce dernier est fixée, par exemple par soudure, l'extrémité inférieure du panier 5 d'écartement et d'extraction. Ce dernier, qui est un extracteur de type connu, présente la double fonction de constituer un panier d'écartement et un panier d'extraction et il est formé d'une pluralité de fils métalliques 5a. Ces derniers suivant un parcours torsadé, par exemple de type hélicoïdal, sont fixés et rassemblés à leurs parties supérieures, avantageusement au moyen d'une soudure à un embout ogival 6. Lorsque les fils 5a sont sortis du cathéter 2, ils retrouvent par suite de leur nature élastique la conformation en forme de panier qui est représentée aux dessins.

L'instrument selon l'invention qui est représenté à la figure 2 présente une conformation analogue à celui de la figure 1 avec cette différence que le panier 5 assume seulement la fonction de panier d'écartement. A l'intérieur de ce panier, vient se positionner le panier intérieur 7, lequel est formé d'une pluralité de fils enroulés en spirales de la même façon que les fils 5a du panier 5. Le panier 7 présente une hauteur qui est sensiblement inférieure par rapport à celle du panier extérieur 5 et l'extrémité supérieure du panier 7 se termine par un embout ogival 8. Indépendamment du sens d'enroulement des fils 5a et 7a, les surfaces d'enveloppement virtuelles des paniers 7 et 5 définissent entre elles une zone ou volume intermédiaire 9 (figure 3), prévu pour recevoir ou emprisonner les excroissances ou aspérités 10a du corps étranger 10 à extraire ; ainsi, ces aspérités 10a ne peuvent venir au contact de la paroi interne 11 du canal physiologique 12, comme cela se produisait dans le cas des instruments de type connu et ainsi qu'on peut le voir sur la figure 1.

6

0169784

Toute possibilité d'entailler ou de léser la paroi interne du canal physiologique se trouve ainsi éliminée et on évite ainsi les interventions chirurgicales éventuelles qui résultaient de lésions consécutives à l'utilisation des appareils antérieurs.

Il résulte clairement de ce qui précède que l'instrument selon l'invention résout efficacement le problème posé et permet d'obtenir les avantages qui ont été précédemment décrits. En particulier, on évite les lésions ou agressions internes tout en conservant la simplicité de construction et les commodités de manipulation des appareils antérieurs. En pratique, la conformation à donner aux fils des paniers comme leur sens de rotation ou leur profil curviligne, tout comme leur nombre, pourront être adaptés ou modifiés à volonté sans s'écarter du domaine de protection de la présente invention. Pareillement la hauteur du palier intérieur pourra être aménagée librement.

En pratique, on a constaté que les meilleurs résultats sont obtenus en adoptant pour le panier intérieur 7 une hauteur sensiblement moitié de la hauteur du panier extérieur 5. Les dimensions et les matériaux (éventuellement sous forme de matériaux stratifiés ou comportant un revêtement) pourront également être modifiés à volonté. L'instrument trouvera son application aussi bien dans le domaine de la médecine que dans le domaine vétérinaire.

## REVENDICATIONS

1 - Instrument pour l'extraction de corps étrangers dans les canaux physiologiques de l'organisme, comportant un tube ou cathéter contenant un câble de commande supportant à son tour un panier formé de fils élastiques, caractérisé en ce que :

a) ledit panier constitue un panier d'écartement extérieur (5) et il contient en son sein un panier intérieur (7) d'extraction conformé de façon analogue ;

b) le panier intérieur d'extraction est relié par son extrémité proximale à l'extrémité proximale du panier d'écartement (5) et l'extrémité distale du panier intérieur d'extraction est indépendante et écartée de l'extrémité distale du panier extérieur (5) ; et

c) entre le panier intérieur d'extraction (7) et le panier extérieur d'écartement (5) se trouve délimité un volume intermédiaire (9) apte à contenir les aspérités ou excroissances (10a) du corps étranger (10) à extraire.

2 - Instrument d'extraction de corps étrangers selon la revendication 1, caractérisé en ce que le panier extérieur d'écartement (5) et le panier intérieur d'extraction (7) présentent le même nombre de fils (5a-7a).

8

3 - Instrument pour l'extraction de corps étrangers selon la revendication 1, caractérisé en ce que le panier extérieur d'écartement (5) et le panier intérieur d'extraction (7) présentent un nombre différent de fils (5a-7a).

4 - Instrument pour l'extraction de corps étrangers selon l'une des revendications 1,2 ou 3 ci-dessus, caractérisé en ce que les fils (5a-7a) du panier extérieur (5) et du panier intérieur (7) sont prévus avec un enroulement dans le même sens.

5 - Instrument pour l'extraction de corps étrangers selon l'une des revendications 1 à 4 dans lequel les fils (5a-7a) du panier extérieur (5) et du panier intérieur (7) sont prévus avec des enroulements de sens contraires.

6 - Instrument pour l'extraction de corps étrangers selon l'une des revendications 1 à 5 ci-dessus, caractérisé en ce que la longueur du panier intérieur (7) est sensiblement égale à la moitié de la longueur du panier extérieur (5).

7 - Instrument pour l'extraction de corps étrangers selon l'une des revendications 1 à 6 ci-dessus, caractérisé en ce que les paniers (5) et (7) sont constitués de fils (5a-7a) présentant des sections transversales différentes.

8 - Instrument pour l'extraction de corps étrangers selon l'une des revendications 1 à 7 ci-dessus, caractérisé en ce que la zone intermédiaire (9) prévue entre la surface d'enveloppement virtuelle des paniers (5-7) présente un volume maximum au niveau de la partie distale du panier intérieur (7).

0169784

Fig:1

Fig:2

Fig:3